(19) Europäisches Patentamt
European Patent Office
Office européen des brevets

(11) Veröffentlichungsnummer: **0 432 619 A2**

# (12) EUROPÄISCHE PATENTANMELDUNG

(21) Anmeldenummer: **90123281.9**

(22) Anmeldetag: **05.12.90**

(51) Int. Cl.5 **G02F 1/35**, C07D 213/38, C07D 213/30

(30) Priorität: **09.12.89 DE 3940712**

(43) Veröffentlichungstag der Anmeldung: **19.06.91 Patentblatt 91/25**

(84) Benannte Vertragsstaaten: **BE DE DK FR GB GR IT NL SE**

(71) Anmelder: **HOECHST AKTIENGESELLSCHAFT**
**Postfach 80 03 20**
**W-6230 Frankfurt am Main 80(DE)**

(72) Erfinder: **Lupo, Donald, Dr.**
**Am Holderbusch 28**
**W-6239 Eppstein/Taunus(DE)**
Erfinder: **Prass, Werner, Dr.**
**Bahnstrasse 19**
**W-6500 Mainz(DE)**
Erfinder: **Scheunemann, Ude, Dr.**
**Feldbergstrasse 12**
**W-6237 Liederbach(DE)**

## (54) Schichtelement und Verfahren zu seiner Herstellung.

(57) Ein für NLO-Zwecke verwendbares Schichtelement läßt sich nach der Langmuir-Blodgett-Technik herstellen. Es besteht aus einem festen Schichtträger und mindestens zwei hierauf aufgebrachten dünnen Schichten regelmäßiger Struktur, die mindestens eine amphiphile Verbindung der allgemeinen Formel (I) aufweisen ,

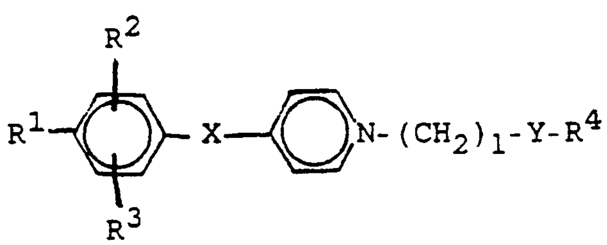

$Z^-$ (I)

wobei $R^1$ $H(CH_2)n^O$, $H(CH_2)n^S$ oder

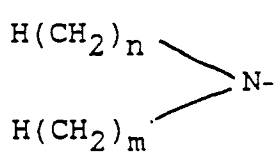

;

$R^2$ und $R^3$, unabhängig voneinander H, $H(CH_2)p^{O-}$, $H(CH_2)p^{S-}$ oder

EP 0 432 619 A2

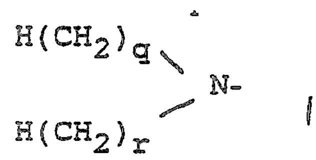

| | |
|---|---|
| X | eine einfache Bindung, -CH=CH-, -N=N-, -CH=N-NH-, oder -CH=N, |
| Y | einen zweiwertigen Rest O, NH oder S, |
| $R^4$ | -H, $-CO-(CH_2)t^H$, $(CH_2)t^{-H}$ |
| | oder |
| $YR^4$ | $-COO^-$, $-CONH_2$, H, OH, $SO_2(CH_2)u^H$, $-CO_2(CH_2)t^H$ oder $-CO_2(CH_2vOH$, |
| Z | mindestens teilweise ein Polyanion , |
| l | eine Zahl von 1-10, |
| n | eine Zahl von 10-25, |
| m | eine Zahl von 0-25, |
| p | eine Zahl von 10-25, |
| q | eine Zahl von 10-25, |
| r | eine Zahl von 0-25, |
| t | eine Zahl von 0-25, |
| u | eine Zahl von 1-20 und |
| v | eine Zahl von 2-10 |

bedeuten.

## SCHICHTELEMENT UND VERFAHREN ZU SEINER HERSTELLUNG

Die vorliegende Erfindung betrifft einen auf einem Träger aufgebrachten Film, der aus mindestens 2 monomolekularen durch ein Polyanion stabilisierten Schichten einer amphiphilen Pyridinium-Verbindung besteht.

Die nichtlineare Optik (NLO) hat eine große Bedeutung für die zukünftige Entwicklung der Informationstechnik, wegen ihres Potentials für schnelle Signalverarbeitung und -Übertragung und für neue Methoden in der Datenverarbeitung. Spezielle organische Verbindungen haben höhere Wirkungsgrade und schnellere Schaltzeiten als die herkömmlichen anorganischen Substanzen für nichtlineare Optik.

Eine Substanz hat nicht-linear-optische Eigenschaften, wenn die Polarisation P, die durch die Wechselwirkung zwischen der Substanz und einem stark elektrischen Feld (einem Laserstrahl oder einem starken Gleichspannungsfeld) erzeugt wird, nach folgender Gleichung von höheren Potenzen der Feldstärke abhängt:

$$\vec{P} = \chi(1) \cdot \vec{E}_1 + \chi(2) : \vec{E}_1\vec{E}_2 + \chi(3) : \vec{E}_1\vec{E}_2\vec{E}_3 + \ldots$$

$\chi(1)$, $\chi(2)$ und $\chi(3)$ sind die sogenannten Suszeptibilitäten 1., 2., und 3. Ordnung. $\vec{E}$ ist das elektrische Feld, das Komponenten mehrerer Frequenzen enthalten kann. Durch NLO-Wechselwirkungen können Felder mit neuen Frequenzen erzeugt sowie die Brechungsindizes des Materials geändert werden. Die Suszeptibilitäten $\chi(2)$ und $\chi(3)$ hängen von den sogenannten molekularen Hyperpolarisierbarkeiten $\beta$ und $\gamma$ ab.

Wichtige nichtlinear-optische Effekte, die von $\chi(2)$ abhängen, sind die Frequenzverdopplung eines Lichtstrahls, insbesondere eines Laserstrahls, die parametrische Verstärkung eines schwachen Lichtsignals und die elektrooptische Umwandlung von elektrischen Signalen. Um Effekte 2. Ordnung zu erzielen, müssen die aktiven Moleküle nichtzentrosymmetrisch ausgerichtet werden, da für zentrosymmetrische Substanzen $\chi(2) = 0$.

Ein Verfahren, mit dem man Filme mit einer für die NLO besonders günstige geordnete Ausrichtung herstellen kann, ist das Langmuir-Blodgett (LB) Verfahren. Bei diesem Verfahren werden Moleküle auf einer Wasseroberfläche gespreitet, durch Verkleinerung der Fläche pro Molekül parallel angeordnet und bei konstantem Schub durch Ein- und Austauchen eines Trägers auf ein Substrat aufgezogen. Pro Tauchgang wird eine monomolekulare Schicht unter Einhaltung ihrer Ordnung übertragen. Für den Aufbau von LB-Schichten verwendet man amphiphile Moleküle, d.h. Moleküle, die ein hydrophiles Ende (einen "Kopf") und ein hydrophobes Ende (einen "Schwanz") haben. Multischichten für den Einsatz in optischen Bauelementen erfordern wiederholte Tauchgänge.

Um LB-Schichten mit hohen Suszeptibilitäten 2. Ordnung zu erzielen, geht man von organischen Verbindungen aus, die sowohl hohe molekulare Hyperpolarisierbarkeiten 2. Ordnung $\beta$ als auch amphiphile Eigenschaften haben. Eine Verbindung hat einen großen Wert für $\beta$, wenn sie ein konjugiertes Elektronensystem (z.B. einen Benzolring) enthält, in das eine oder mehrere Elektronendonorgruppen und eine oder mehrere Elektronenakzeptorgruppen eingebaut werden. Am Donor- oder Aktzeptorende wird eine hydrophobe Gruppe eingebaut. Die Hyperpolarisierbarkeit wird vergrößert, wenn das Molekül Licht im Wellenlängenbereich des einstrahlenden elektrischen Feldes oder des durch die NLO erzeugten Feldes absorbiert (sogenannte Resonanzverstärkung). Allerdings sind Absorptionen für viele Anwendungen unerwünscht, da sie Verluste verursachen und die optische Stabilität (die Lichtintensität, die ohne permanente Materialstörungen ertragen werden kann) negativ beeinflussen. Eine ideale Verbindung hat eine große Hyperpolarisierbarkeit, ohne im gewünschten Wellenlängenbereich stark zu absorbieren.

Es war schon bekannt, daß die Pyridinium-Verbindung der allgemeinen Formel Ia

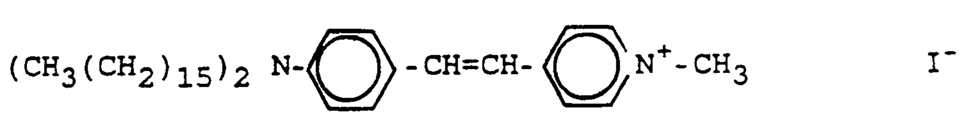

(Ia)

und ähnliche Verbindungen sehr gute NLO-Eigenschaften in Monoschichten aufweisen (Lupo et al., J Opt. Soc. Amer., B 5, 500 (1988)). Mit diesen Pyridinium-Verbindungen oder ähnlichen Farbstoffen, die

einwertige Anionen enthalten, läßt sich üblicherweise höchstens eine Doppelschicht aufziehen; weitere Tauchgänge führen nicht zur weiteren Übertragung des Farbstoffs. Ferner sind die Schichten der Farbstoffe mit einwertigem Anion üblicherweise nur bis 70° C stabil,

Es bestand daher die Aufgabe, einen Film aus amphiphilen Pyridinium-Verbindungen so zu stabilisieren, daß nacheinander mehrere Monoschichten unter Bildung von Multischichten übertragen werden können. Gleichzeitig sollte nach Möglichkeit die thermische Stabilität (im Vergleich zu den bekannten Filmen) verbessert werden.

Es wurde nun ein Schichtelement gefunden, das aus einem festen Schichtträger und mindestens zwei hierauf aufgebrachten dünnen Schichten regelmäßiger Struktur besteht, die mindestens eine amphiphile Verbindung der allgemeinen Formel (I) enthalten

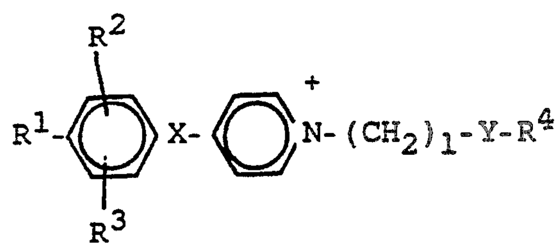

$Z^-$ (I)

wobei $R^1$ $H(CH_2)n^{O}$, $H(CH_2)n^{S}$ oder

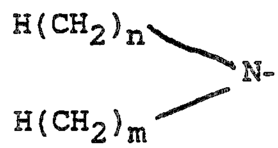

$R^2$ und $R^3$, unabhängig voneinander
H, $H(CH_2)p^{O-}$, $H(CH_2)p^{S-}$ oder

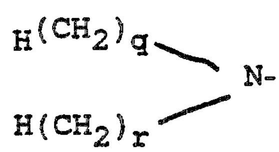

X eine einfache Bindung, -CH = CH-, -N = N-, -CH = N-NH-,
oder -CH = N-, vorzugsweise -CH = CH, -N = N oder -CH = N-NH-, Y einen zweiwertigen Rest O, NH oder S,
$R^4$ H, $-CO(CH_2)t^{H,}$ $-(CH_2)t^{-H}$
oder

$YR^4$ $-COO^-$, $-CONH_2$, H, OH, $-SO_2(CH_2)u^{H}$, $-CO_2(CH_2)t^{H}$ oder $-CO_2(CH_2)v^{OH}$,

Z mindestens teilweise ein Polyanion,
1 eine Zahl von 1-10, vorzugsweise 1- 3,
n eine Zahl von 10-25, vorzugsweise 14-24,
m eine Zahl von 0-25, vorzugsweise 14-24,
p eine Zahl von 10-25, vorzugsweise 14-24,
g eine Zahl von 10-25, vorzugsweise 14-24,
r eine Zahl von 0-25, vorzugsweise 14-24,
t eine Zahl von 0-25, vorzugsweise 0- 5, insbesondere 1-5, besonders bevorzugt 1-3,
u eine Zahl von 1-20, und
v eine Zahl von 2-10, vorzugsweise 2-5 bedeuten.

Der Pyridiniumring kann am N-Ende oder C-Ende der Reste -NH-N = CH- und -N = CH- angeordnet sein.

Das Polyanion Z leitet sich von Säuren ab, die mindestens 10 saure Gruppen im Molekül enthalten.

Vorzugsweise enthält die saure Verbindung jedoch mehr als 20 saure Gruppen im Molekül, insbesondere mehr als 50 saure Gruppen. Beispiele für anorganische Säuren, die Polyanionen liefern, sind Metaphosphorsäure oder Polyphosphorsäure. Bevorzugt sind organische Polysulfonsäuren, wie Polyvinylsulfonsäure und Polystyrol-Sulfonsäure und Polycarbonsäuren wie Polyacrylsäure oder Polymethylacrylsäure. Verwendbar sind auch Polycyanacrylsäure und Polyfluoracrylsäure. Das Molekulargewicht der Polysäure ist nicht entscheidend, sofern der Polymerisationsgrad mindestens 10 beträgt. Es ist nicht erforderlich, daß dem Pyridiniumkation in der Schicht ausschließlich das Polyanion gegenübersteht; es reicht vielmehr aus, wenn mindestens 50% der erforderlichen negativen Ladungen, vorzugsweise mindestens 90%, durch Polyanionen geliefert werden; der Rest kann auf beliebige, insbesondere wasserlöslich machende Anionen, z.B. einwertige Anionen, wie Halogenid, Monomethylsulfat, Hydrogensulfat, Perchlorat, Nitrat entfallen. Acetat und Propionat, insbesondere Sulfonate wie Toluolsulfonat, erhöhen die Löslichkeit der Salze in organischen Lösungsmitteln. Die entsprechenden Pyridiniumsalze mit Polyanionen sind meist in Wasser unlöslich.

Die erfindungsgemäßen Schichtelemente lassen sich dadurch herstellen, daß man mindestens eine amphiphile Verbindung der allgemeinen Formel (II)

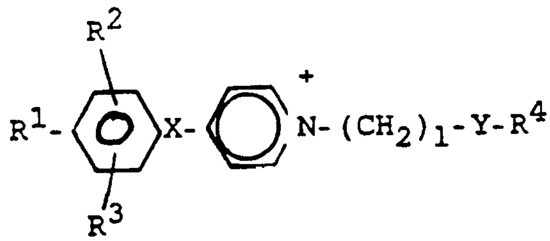

$Z'^{-}$ (II)

wobei $R^1$ $H(CH_2)n^{O-}$ $H(CH_2)n^{S}$ oder

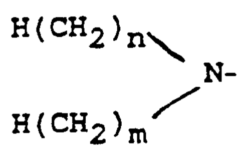

$R^2$ und $R^3$, unabhängig voneinander H, $H(CH_2)p^{O-}$, $H(CH_2)p^{S-}$ oder

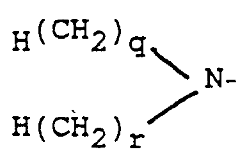

,

| | |
|---|---|
| X | eine einfache Bindung, -CH = CH-, -N = N-, -CH = N-NH-, oder -CH = N-, |
| Y | einen zweiwertigen Rest O, NH oder S, |
| $R^4$ | H, $-CO-(CH_2)t^H$, $-(CH_2)t^{-H}$ oder |
| $YR^4$ | $-COO^-$, $-CONH_2$, H, OH, $-SO_2(CH_2)u^H$, $-CO_2(CH_2)t^H$ oder $-CO_2(CH_2)v^{OH-}$ |
| Z' | ein löslich machendes Anion, |
| l | eine Zahl von 1-10, vorzugsweise 1- 4, |
| n | eine Zahl von 10-25, vorzugsweise 14-24, |
| m | eine Zahl von 0-25, vorzugsweise 14-24, |
| p | eine Zahl von 10-25, vorzugsweise 14-24, |
| q | eine Zahl von 10-25, vorzugsweise 14-24, |
| r | eine Zahl von 0-25, vorzugsweise 14-24, |
| t | eine Zahl von 0-25, vorzugsweise 0- 5, insbesondere 1-5, |
| u | eine Zahl von 1-20 und |

v eine Zahl von 2-10, vorzugsweise 2-5

bedeuten,

in einem flüchtigen, mit Wasser nicht mischbaren Lösungsmittel löst, man die Lösung auf eine Phasengrenzfläche Luft/Wasser aufbringt, wobei das Wasser ein Polyanion $Z^-$ enthält, man die nach Verdunsten des Lösungsmittels zurückbleibende Schicht komprimiert und nach der Langmuir-Blodgett-Technik auf einen festen Schichtträger überträgt. Vorzugsweise werden Pyridiniumsalze eingesetzt, die am Phenylring zwei Alkylketten mit 14 bis 24 C-Atomen an $R^1$ (eine Dialkylamino-Gruppe) oder $R^1$ und $R^2$ (Alkoxy- und/oder Alkylthio-Gruppen tragen).

Man kann auch die Salze der Formel II zusammen mit einer zweiten amphiphilen Verbindung einsetzen. Dabei soll der Anteil der zweiten amphiphilen Verbindung 0-60, vorzugsweise 0-10, Gew.-% betragen. Ebenso können Schichten mit einem Gehalt an Pyridiniumsalzen I abwechseln mit Schichten aus Molekülen der zweiten amphiphilen Verbindung. Dies ist insbesondere hilfreich, um sicherzustellen, daß die Moleküle des Salzes (I) nicht-zentrosymmetrisch orientiert sind.

Der hydrophobe Teil der zweiten amphiphilen Verbindung soll eine gewisse Mindestlänge aufweisen. Es ist bevorzugt, wenn die zweite amphiphile Verbindung mindestens einen hydrophoben Teil, in dem mindestens 8 Kohlenstoffatome enthalten sind, und mindestens eine polare Äther-, Hydroxy-, Carbonsäure-, Carbonsäureester-, Amin-, Carbonsäureamid-, Ammoniumsalz-, Sulfat-, Sulfonsäure-, Phosphorsäure-, Phosphonsäure-, Phosphonsäureester-, Phosphonsäureamid-, Phosphorsäureester- oder Phosphorsäureamid-Gruppe enthält.

Besonders bevorzugt ist es, wenn die amphiphile Verbindung aus mindestens einem hydrophoben Teil mit mindestens 8 Kohlenstoff-Atomen und mindestens einem polaren Teil besteht, der ausgewählt ist aus den folgenden Gruppen

$-OR^8$

$-COOR^6$

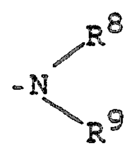

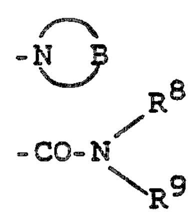

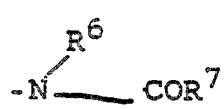

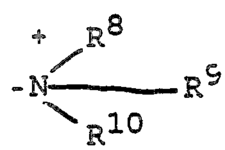

- $SO_3H$
- $OSO_3R^6$
- $OPO(OR^6)(OR^7)$
- E

- O-E
- $NR^6$-E,

wobei $R^6$ bis $R^{10}$, B und E folgendes bedeuten:

| | |
|---|---|
| $R^6$ und $R^7$ | unabhängig voneinander H oder $C_1$-$C_3$-Alkyl, |
| $R^8$, $R^9$ und $R^{10}$ | unabhängig voneinander H, $C_1$-$C_4$-Alkyl, -$C_2H_4OH$ oder -$CH_2$-CHOH-$CH_3$, insbesondere H oder $CH_3$ |
| B | einen zweiwertigen organischen Rest, so daß -NB einen stickstoffhaltigen Heterocyclus, insbesondere einen 5- oder 6-gliedrigen, gesättigten oder ungesättigten Heterocyclus mit 1 bis 3 C-Atomen oder N- und O-Atomen oder N- und S-Atomen bildet, und |

E

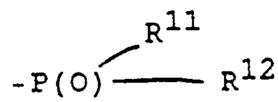

oder

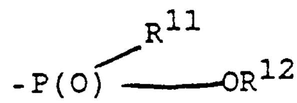

,

wobei $R^{11}$ und $R^{12}$ unabhängig voneinander für

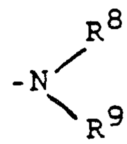

stehen.

Beispielsweise kann die amphiphile Verbindung eine Fettsäure der allgemeinen Formel $CH_3(CH_2)_gCO_2H$ sein,
wobei g eine Zahl von 8 bis 25, vorzugsweise 12 bis 22, bedeutet.

Vorteilhafterweise wird als zweite amphiphile Verbindung ein ungesättigtes Säureamid der allgemeinen Formel III

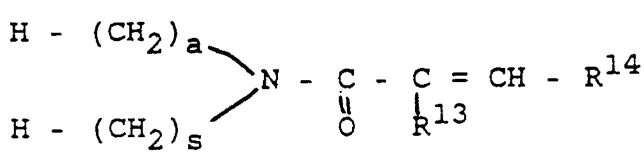

(III)

eingesetzt, in der

| | |
|---|---|
| $R^{13}$ H, | Cl, F, CN oder $(CH_2)_bH$, |
| $R^{14}$ H | , $(CH_2)_cH$ oder -CH=CH-$(CH_2)_cH$, |
| a und s | unabhängig voneinander eine Zahl von 0 - 22 und |
| b und c | unabhängig voneinander eine Zahl von 0 - 24 insbesondere 0-18, bedeuten. |
| a ist | vorzugsweise eine Zahl von null bis 18 und s ist vorzugsweise null. |

Die Konzentration des Polyanions in der wäßrigen Sub-Phase ist in weiten Grenzen wählbar, da bei der Übertragung des Films nur winzige Mengen Polyanion verbraucht werden. Verwendbar sind beispielsweise Konzentrationen von $1x10^{-6}$ bis $1,5x10^{-2}$ Säureäquivalent/L.

Das löslich machende Anion spielt für das Spreiten keine Rolle.

Die Moleküle werden bei der Langmuir-Blodgett-Technik mit einer Barriere zusammengeschoben, wobei die Alkylkette bei wachsender Oberflächendichte im wesentlichen senkrecht zur Grenzschicht orientiert werden. Während der Kompression entsteht durch Selbstorganisation der Moleküle an der Grenzschicht ein

hochgeordneter monomolekularer Film, dessen konstante Schichtdicke im wesentlichen durch die Kettenlänge der Alkylseitenketten der Polymeren und deren Tiltwinkel (das ist der Winkel, um den die Molekülketten auf der Wasseroberfläche gegen die Normale gekippt sind) bestimmt wird. Die typische Dicke eines solchen Films liegt bei 2 - 3 nm.

Aus der Abmessung der Oberfläche, dem Spreitvolumen und der Konzentration der Lösung läßt sich die mittlere Fläche pro Molekül berechnen. Phasenübergänge bei der Kompression der Moleküle lassen sich in der Schub-Flächen Isotherme erkennen.

Der Film wird bei konstantem Schub durch Eintauchen oder Austauchen eines geeigneten Trägers unter Erhalt der Ordnung von der Wasseroberfläche abgenommen.

Als Subphase für die Monofilmherstellung dient meist eine Lösung der Polysäure oder deren Salze in Wasser. Anstelle von Wasser sind aber auch andere Flüssigkeiten mit hoher Oberflächenspannung, wie z.B. Glycerin, Glykol, Dimethylsulfoxid, Dimethylformamid oder Acetonitril verwendbar, in denen die Polysäure als Säure oder Salz - nicht aber das Pyridiniumsalz - löslich ist.

Als Träger kommen beliebige feste, vorzugsweise dimensionsstabile Substrate aus unterschiedlichen Materialien in Betracht. Die als Schichtträger dienenden Substrate können beispielsweise transparent oder lichtdurchlässig, elektrisch leitend oder isolierend sein.

Das Substrat kann hydrophob oder hydrophil sein. Die Oberfläche des Substrats, auf die die LB-Schicht aufgebracht wird, kann hydrophobiert sein. Die zu beschichtende Oberfläche des Substrats sollte möglichst rein sein, damit die Ausbildung einer dünnen, geordneten Schicht nicht gestört wird. Insbesondere die Anwesenheit von oberflächenaktiven Stoffen auf der zu beschichtenden Oberfläche der Substrate kann die Schichtherstellung beeinträchtigen. Es ist möglich, die als Schichtträger dienenden Substrate auf der zu beschichtenden Oberfläche vor dem Aufbringen der LB-Filme zunächst mit einer Zwischenschicht zu versehen, um z.B. die Haftung des Films auf dem Substrat zu verbessern.

Als Materialien für die Substrate können beispielsweise Metalle, wie etwa Gold, Platin, Nickel, Palladium, Aluminium, Chrom, Niob, Tantal, Titan, Stahl und dergleichen verwendet werden. Andere geeignete Materialien für die Substrate sind Kunststoffe, wie beispielsweise Polyester, etwa Polyethylenterephthalat oder Polybutylenterephthalat, Polyvinylchlorid, Polyvinylidenchlorid, Polytetrafluorethylen, Polystyrol, Polyethylen oder Polypropylen.

Gleichermaßen kommen auch Halbleiter, wie Silizium, Germanium oder Galliumarsenid oder auch Glas, Siliziumdioxid, keramische Werkstoffe oder Celluloseprodukte für die Substrate in Betracht. Die Oberfläche von Glas und anderen hydrophilen Substraten kann, sofern erforderlich, in an sich bekannter Weise durch Umsetzung mit Alkylsilanen oder Hexamethyldisilazan hydrophobiert werden. Die Auswahl der Substratmaterialien richtet sich in erster Linie nach dem Verwendungszweck der aus dem erfindungsgemäßen Film hergestellten Schichtelemente. Werden die erfindungsgemäßen Schichtelemente beispielsweise in der Elektronik oder bei elektrochemischen Prozessen verwendet, dienen als Substrate insbesondere elektrisch leitfähige Materialien, wie Metalle oder metallische Oberflächenschichten, beispielsweise auf Kunststoffolien oder Glas.

Die als Träger für die erfindungsgemäßen Filme dienenden Substrate können, je nach Verwendungszweck, beliebige Formen aufweisen. Beispielsweise können sie film-, folien-, platten-, bandförmig oder auch zylinderförmig sein oder unter anderen beliebigen Formen ausgewählt werden. Im allgemeinen wird es sich bei den Schichtträgern um flache, ebene Substrate handeln, wie z.B. Filme, Folien, Platten, Bänder und dergleichen. Die zu beschichtende Oberfläche der Substrate ist vorzugsweise glatt, wie es für die Herstellung von LB-Filmen üblich ist. Bei flachen, ebenen Substraten können die erfindungsgemäßen Filme auf eine oder beide Oberflächen des Substrats aufgebracht werden.

Der erfindungsgemäße Film ergibt eine stabile Multischicht mit guten nichtlinear-optischen Eigenschaften und guter thermischer Stabilität. In einer Multischicht ist er dadurch beispielsweise für elektrooptische Schalter, Diodenlaserfrequenzverdoppler oder optische Verstärker geeignet.

Die erfindungsgemäßen Schichtelemente lassen sich auch für optische Zwecke einsetzen. Dabei ist es von Vorteil, daß das Absorptionsmaximum der LB-Schicht gut beeinflußt werden kann. Von großem Einfluß ist hierfür die Struktur der mit dem Pyridiniumring verknüpften Gruppe X. In der Reihenfolge Einfachbindung $<$ -HC=CH- $<$ -N=CH- $<$ -N=N-CH- $<$ -N=N-wird dabei die Wellenlänge des Absorptionsmaximums immer größer und dieses schließlich in den sichtbaren Bereich verschoben. Für den Einsatz der erfindungsgemäßen Schichtelemente ist Farbigkeit nicht erforderlich. Für die Frequenzverdopplung von Diodenlaserstrahlung ist es günstig, wenn Bereich von 400-800 nm keine Lichtabsorption erfolgt.

Für den Sonderfall x = -CH=CH- ist der Einfluß der Substituenten $R^1$ und $(CH_2)_1$-Y-$R^4$ auf das Absorptionsmaximum des Films aus der Tabelle 1 abzulesen.

Die kationischen Verbindungen der Formel I sind entweder bekannt oder lassen sich leicht herstellen. Schiffsche Basen (X-bedeutet -CH=N-) lassen sich in bekannter Weise aus Aldehyden und Aminen

herstellen. Sie zersetzen sich aber langsam an feuchter Luft, noch schneller auf einer Wasseroberfläche. Synthesewege zur Herstellung der N-Methyl-Verbindungen (1 = 1; $YR^4$ = H) mit x = -CH = CH (Schema 1, 2), x = -CH = N - N - (Schema 3), x = -N = N (Schema 4) und x = Einfachbindung (Schema 5) sind am Ende der Beschreibung skizziert.

Dabei erfolgte die Quaternierung des Pyridin-Ringes mit Methyljodid (1 = 1; $YR^4$ = H). Analog ist auch eine Quaternierung mit anderen Reagenzien, wie 3-Brompropanol, Chloressigsäureäthylester oder 3-Brompropionsäure möglich.

Es war bekannt (M. Shimomura, K. Fuhjii, P. Karg, W. Frey, E. Sackmann, P. Meller, H. Ringsdorf, Jap. J. Appl. Phys. 27, 1988, L7161-7163), daß Monofilme aus kationischen Amphiphilen ohne Chromophor, wie

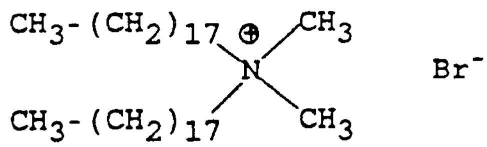

ein verbessertes Übertragungsverhalten bei der Langmuir-Blodgett-Technik zeigen, wenn in der wäßrigen Subphase Polyanionen vorhanden sind, die sich z.B. ableiten von Polyvinylsulfonsäure und Polystyrolsulfonsäure.

Monofilme mit Polyanionen zeigen ein besseres Übertragungsverhalten, eine höhere Stabilität und eine bessere Ausrichtung der Chromophore als Filme mit einwertigen Aminen. Eine gute Ausrichtung ist wichtig für die Güte der erreichbaren Frequenzverdopplung bei Einsatz in der NLO-Technik. Wie sich aus dem Verlauf der Abhängigkeit der Intensität der Frequenzverdopplung vom Einfallswinkel ableiten läßt, wird durch das Polyanion die Ausrichtung der Chromophore um etwa 5° "steiler".

Die Erfindung wird durch die nachfolgenden Beispiele näher erläutert.

Beispiel 1

Schichtherstellung nach der Langmuir-Blodgett Methode

Glas-Objektträger (76 mm x 26 mm) werden nach folgendem Verfahren gereinigt: Das Glas wird eine Stunde lang in eine 60° C warme, frisch angesetzte Mischung aus vier Teilen konz. $H_2SO_4$ und einem Teil 30%iger $H_2O_2$ gelegt, mit sauberem Wasser abgespült und 15 Minuten lang in einer Reinigungslösung (Extran® AP 11, Konz. 2-4 g/l) bei 50° C ultrabeschallt. Danach wird wieder gründlich mit sauberem Wasser abgespült und in einem warmen Luftstrom getrocknet. Anschließend erfolgt zur Hydrophobierung eine Behandlung mit Hexamethyldisilazan-Dampf (10 Minuten bei 70° C).

Der auf Seite 3 erwähnte Farbstoff der allgemeinen Formel Ia wird in Methylenchlorid gelöst. Die Lösung wird auf der wäßrigen Subphase in einer Langmuir-Filmwaage gespreitet. Als Subphasen werden reines Wasser sowie wäßrige Lösungen von Polyacrylsäure, durch Zugabe von NaOH auf pH 6.0 gebracht, in Konzentrationen von 0.01 mg/l bis 1000 mg/l verwendet. In einem Konzentrationsbereich von 0.1 mg/l bis 100 mg/l werden Multischichten nach der Langmuir-Blodgett-Methode auf einem Glasträger durch Übertragung erzeugt:

Durch Verkleinerung der monofilmbedeckten Wasseroberfläche wird der Schub auf 30 mN/m eingeregelt und bei diesem Wert konstant gehalten. Der Träger wird nun senkrecht von oben durch die Wasseroberfläche in die Filmwaage eingetaucht (Eintauchgeschwindigkeit: 200 mm/min.) und nach einer kurzen Pause (10 sec.) am unteren Umkehrpunkt wieder herausgenommen (Austauchgeschwindigkeit: 10mm/min.). Sowohl beim Eintauchals auch bei Austauchvorgang wird dabei eine Monolage auf den Träger übertragen. Durch mehrfache Wiederholung des Tauchvorgangs nach jeweils eine Minute Wartezeit am oberen Umkehrpunkt werden in Gegenwart des Polyanions ohne Schwierigkeiten 20 Doppelschichten übertragen. Die Übertragungsverhältnisse liegen zwischen 80 und 100%. Mit reinem Wasser als Subphase können nur 2 Doppel-Lagen mit Übertragungsverhältnissen unter 50 % erhalten werden.

Nach dem gleichen Verfahren wurden auch Schichten der Pyridiniumsalze der Formeln

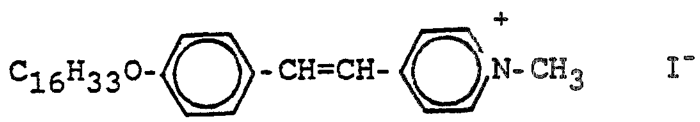 (Ib)

und

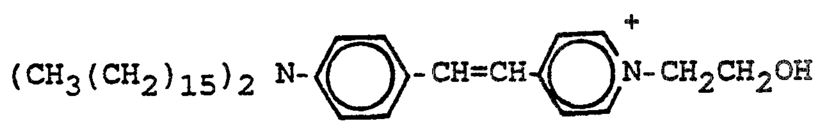 (Ic)

hergestellt. Dabei betrug die Subphasen-Temperatur 20° C, der Schub 30 mN/m.
Ergebnis: Es wurden klar durchsichtige, farbige Multischichten erhalten. Die Übertragungsverhältnisse liegen zwischen 80 und 100 %.

Beispiel 2
Messungen der thermischen Stabilität

Siliziumplättchen (40 mm x 10 mm) werden aus einem thermisch oxidierten Siliziumwafer (Dicke der Oxidschicht: 100 nm) herausgeschnitten und eine Stunde lang bei 60° C in eine frisch zubereitete Mischung aus einem Teil 30%igem $H_2O_2$ und vier Teilen konzentrierter Schwefelsäure gelegt. Nach gründlichem Abspülen mit sauberem Wasser werden die Plättchen 15 Minuten lang in einem Ultraschallbad mit alkalischer Reinigungsflüssigkeit (Extran® APII, Konz. 2-4 g/l) behandelt, mit sauberem Wasser gründlich abgespült und in einem warmen Luftstrom getrocknet. Danach erfolgt zur Hydrophobierung eine Behandlung mit Hexamethyldisilazan-Dampf (10 Minuten bei 70° C).

Die Beschichtung nach der LB-Methode mit jeweils 8 Monolagen erfolgt mit den in Beispiel 1 beschriebenen Substanzen nach dem in Beispiel 1 beschriebenen Verfahren.

Der beschichtete Träger wird in einer speziellen Apparatur mit linearem Temperaturgradienten (0,5° C/sec.) aufgeheizt. Während des Aufheizvorgangs wird die Dicke der LB-Schicht anhand der Intensität eines von der Probe reflektierten, senkrecht polarisierten Laserstrahl (633 nm) gemessen. Die Temperatur, bei der die erste Änderung der Schichtdicke erfolgt, beträgt 100° C. (Zum Vergleich: Bei LB-Schichten aus 22-Tricosensäure beträgt diese Temperatur 70° C).

Beispiel 3
Messungen der kritischen Oberflächenspannung

Silizumplättchen (40 mm x 10 mm) werden nach folgendem Verfahren gereinigt: Eine Stunde Behandlung in einem Ultraschallbad in einer Mischung aus einem Teil 30%igen $H_2O_2$ und vier Teilen konzentrierter Schwefelsäure, anschließend Abspülen mit sauberem Wasser. Dann werden die Plättchen 20 Sekunden lang in eine mit Ammoniumfluorid gepufferte HF-Lösung getaucht und anschließend mit sauberem Wasser abgespült. Nach dieser Behandlung sind sie hydrophob.

Die Siliziumplättchen wurden mit acht Monolagen der in Beispiel 1 verwendeten Substanz Ia nach der dort angegebenen Methode beschichtet (Konzentration an Polyacrylsäure in Subphase 10 mg/L).

Auf die Oberfläche der übertragenen Schichten werden eine Reihe flüssiger n-Alkane ($C_9H_{20}$ - $C_{16}H_{34}$) aufgetropft und die Kontaktwinkel der Flüssigkeitstropfen-mit der Oberfläche gemessen. Aus diesen Kontaktwinkeln wird nach dem Verfahren von Zisman die kritische Oberflächenbespannung bestimmt.

Sie beträgt hier 20-22 mN/m.
(Zum Vergleich: Bei einer Polyethylen-Oberfläche ergibt sich bei dieser Messung ein Wert von 31 mN/m).

Beispiel 4
Bestimmung der nichtlinear-optischen Eigenschaften

Glas-Objektträger (76 x 26 mm) wurden nach dem in Beispiel 2 beschriebenen Verfahren gereinigt, ohne den Hydrophobierungsschritt durchzuführen. Die so behandelten Objektträger waren dann hydrophil. Die Träger wurden in einer Langmuir-Filmwaage in Subphasen aus wäßrigen Lösungen von Polyacrylsäure (1 mg/l und 5 mg/l) eingetaucht, die in Beispiel 1 beschriebenen Substanzen an der Wasseroberfläche gespreitet, komprimiert und die Glasträger aus der Subphase bei einem Schub von 30 mN/m und einer

Geschwindigkeit von 1 cm/min. bei 20° C herausgezogen. Auf diese Weise wurde eine Monoschicht auf den Glasträger übertragen.

Den Wert der Suszeptibilität 2. Ordnung $\chi(2)$ wurde nach der Methode der optischen Frequenzverdopplung gemessen, unter Anwendung folgender experimenteller Anordnung:

Ein Nd: YAG Laser erzeugt einen gepulsten Laserstrahl (Pulsdauer ca. 30 ps) mit Wellenlänge 1064 nm ($\omega$ = 9398 $cm^{-1}$), der mittels eines Strahlteilers in einen Referenz- und einen Probenstrahl geteilt wird. Der Referenzstrahl wird durch Frequenzverdopplung in einer Referenzprobe bestehend aus einem polykristallinen Pulver einer organischen Verbindung mit hohem $\chi(2)$ in eine Referenzharmonische mit Wellenlänge 532 nm umgewandelt, deren Intensität von einem Fotodetektor gemessen wird. Der Probestrahl bestrahlt die auf eine Seite des Glasträgers übertragene Langmuir-Blodgett-Monoschicht, die senkrecht zur optischen Ebene auf einem Drehtisch montiert ist. Die Probe wird um den Einfallswinkel gedreht. Der frequenzverdoppelte Strahl wird von einem Fotodetektor gemessen, seine Intensität durch Bezugsnahme auf das Referenzsignal normiert, und die Oberwellenintensität als Funktion des Einfallswinkels gemessen. Die Intensität der Probe wird danach geeicht durch Vergleich mit der harmonischen Welle einer geeichten Quarzplatte. Aus der Größe und Winkelabhängigkeit der Oberwellenintensität können die Nichtlinearität ($\chi(2)$) und die durchschnittliche Orientierung der Chromophore (Tilt-winkel gegen Substratnormale) bestimmt werden.

Folgende Werte für die Nichtlinearität wurden bestimmt:

| Konzentration Polyacrylsäure | $\chi(2)$ | Tiltwinkel |
|---|---|---|
| 0 mg/L | 250 pm/V | 32° |
| 1 | 270 | 27° |
| 5 | 205 | 27° |

(Zum Vergleich: Die Suszeptibilität der kommerziell in Form von Kristallen verwendeten Verbindung $LiNbO_3$ beträgt ca. 8 pm/V).

Beispiel 5 (Vergleichsbeispiel)

Ein Glas-Objektträger wird wie in Beispiel 1 gereinigt und hydrophobiert.

Farbstoff Ia wird aus einer Lösung in Methylenchlorid (Konzentration: 1 mg/ml) auf einer wäßrigen Subphase gespreitet. Als Subphasen werden wäßrige Lösungen von Zitronensäure, Oxalsäure und Schwefelsäure bei einer Konzentration von 5 mg/l eingesetzt, die durch Zugabe von NaOH auf pH 7 gebracht wurden. Die Versuche zur Beschichtung nach der Langmuir-Blodgett Methode erfolgten wie in Beispiel 1 beschrieben. Die Ergebnisse der Versuche sind in folgender Tabelle aufgelistet:

| | | | |
|---|---|---|---|
| Subphase: | Zitronensäure | Oxalsäure | Schwefelsäure |
| Subphasentemp.: | 10, 20 °C | 20 °C | 20 °C |
| Schub: | 30 mN/m | 20, 30 mN/m | 30 mN/m |
| Übertragunsverh.: | Übertragung von 3-Monolagen bei Übertragungsverhältnissen unter 50 % | Übertragung von 3 Monolagen bei Übertragungsverhältnissen unter 50 % | Übertragung von 4 Monolagen bei Übertragungsverhältnissen um 50 % |

Beispiel 6
Synthese vom Farbstoff I a
Quaternierung von 4-Picolin mit Methyliodid

4,85 ml (4,66 g, 50 mmol) 4-Picolin werden bei 20° C in einem Glaskolben vorgelegt und unter Kühlung 3,11 ml (7,1 g, 50 mmol) Methyliodid vorsichtig zudosiert. Nach beendeter Zugabe wird der entstandene Feststoff in 80 ml trockenem Aceton aufgenommen und eine Stunde am Rückfluß gekocht. Anschließend läßt man in der Tiefkühltruhe auskristallisieren und kristallisiert noch einmal aus Ethanol um. Man erhält 5.99 g (25,5 mmol, 51 %) eines weißen Pulvers.

$^1$H-NMR (100 MHz, $CDCl_3$) :$\delta$ = 2,70 (s, 3H, $-CH_3$); 4,65 (s, 3H, $N-CH_3$); 7,75-7,95 (d, 2H, aromat. H); 9,1-9,3 (d, 2H, aromat. H)

Alkylierung von Anilin mit 1-Bromhexadecan

23,3 g (0,25 mol) frisch destilliertes Anilin und 168 g (0,55 mol) 1-Bromhexadecan werden in einen Einhalskolben mit Rückflußkühler und unter Argonatmosphäre 3 Tage bei 90° C gerührt. Nach dem Abkühlen werden 0,7 1 Toluol zugegeben und die Lösung gegen eine 10 prozentige wäßrige Lösung von $Na_2CO_3$ ausgeschüttelt. Durch Ausschütteln mit einer fünfprozentigen wäßrigen HCl-Lösung wird das Hydrochlorid ausgefällt und über eine Nutsche abfiltriert. Anschließend wird die freie Base durch Zugabe von 10 % $Na_2CO_3$ freigesetzt, 0,7 1 Toluol zugegeben, die organische Phase abgetrennt und mit $Na_2SO_4$ getrocknet und das Lösungsmittel im Vakuum entfernt. Zum Schluß wird das Produkt noch zwei Mal aus je 1,5 l Ethanol umkristallisiert. Man erhält 56,4 g (0,1 mol, 42 %) eines weißen Pulvers, das zwischen 47,5 und 49° C schmilzt.

$^1$H-NMR (100 MHz, $CDCl_3$): $\delta$ = 0,7-1,0 (t, 6H, $-CH_3$); 1,1-1,4 (m, 56H, $-CH_2$-Alkyl); 3,1-3,3 (t, 4H, $N-CH_2$); 6,5-6,8, 7,0-7,3 (m, 5H, aromat. H)

Vilsmeier-Formylierung von N,N-Dihexadecylanilin

10 g (18,5 mmol) N,N-Dihexadecylanilin werden mit 75 ml trockenem Dimethylformamid angeteigt, auf 5° C abgekühlt und innerhalb von fünf Minuten mit 2,8 g (18,5 mmol) frisch destilliertem $POCl_3$ versetzt. Danach läßt man auf 20° C erwärmen, rührt eine Stunde bei dieser Temperatur und erhitzt für drei Stunden auf 80° C. Nach dem Abkühlen der Reaktionslösung zersetzt man die Reaktionsmischung mit 40 g Eiswasser und neutralisiert mit ca. 10 ml 5 molarer NaOH-Lösung. Der entstandene Niederschlag wird abgesaugt und aus Ethanol umkristallisiert. Man erhält 4,71 g (8,3 mmol, 45 %) eines cremefarbenen Pulvers.

$^1$H-NMR (100 MHz, $CDCl_3$): $\delta$ = 0,7-1,0 (t, 6H, $-CH_3$); 1,1-1,4 (m, 56H, $-CH_2$-Alkyl); 3,15-3,4 (t, 4H, $N-CH_2$); 6,7-6,9 (d, 2H, aromat. H); 7,65-7,85 (d, 2H, aromat. H); 9,75 (s, 1H, CHO)

Umsetzung von 4-[N,N-Dihexadecyl]aminobenzaldehyd mit N-Methyl-4-picoliniumiodid

0,94 g (4 mmol) N-Methyl-4-picoliniumbromid, 2,28 g (4 mmol) 4-[N,N-Dihexadecyl]aminobenzaldehyd und 1,5 ml Piperidin werden in 150 ml Ethanol suspendiert und 3 Stunden am Rückfluß gekocht. Man läßt

abkühlen und in der Tiefkühltruhe auskristallisieren. Das Rohprodukt wird säulenchromatographisch gereinigt an Kieselgel Si 60 (Laufmittel: $CH_2Cl_2/CH_3OH$ 20:1). Man erhält 1,5 g (1,9 mmol, 48 %) eines roten Pulvers, das bei Temperaturen über 200° C unter Zersetzung schmilzt.

$^1$H-NMR (100 MHz, $CDCl_3$): $\delta$ = 0,7-1,0 (t, 6H, $-CH_3$); 1,1-1,4 (m, 56H, $-CH_2$-Alkyl); 3,15-3,4 (t, 4H, N-$CH_2$); 4,3 (s, 3H, N-$CH_3$); 7,1 (m, 6H, aromat. H); 8,65 (m, 2H, aromat. H)

Tabelle 1

| Strukturformel | Absorption in LB-Schicht $\lambda_{max}/nm$ . |
|---|---|
| 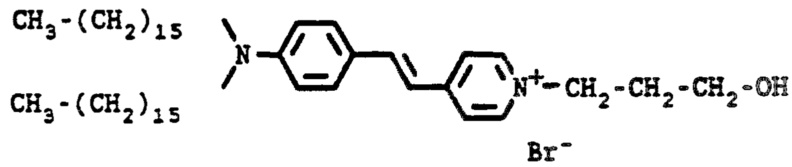 | 475 |
| 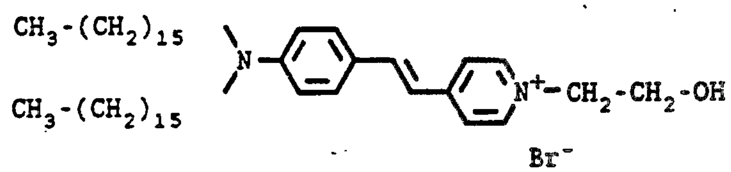 | 460 |
| 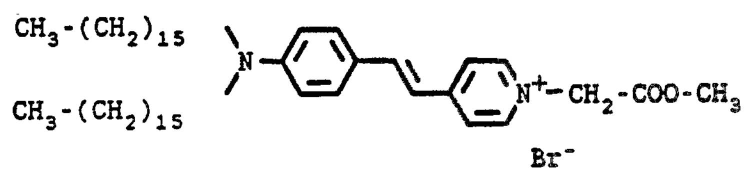 | 470 |
| 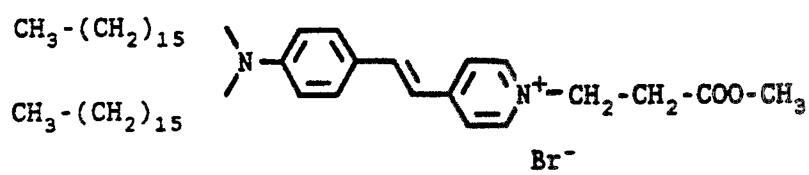 | 475 |
| 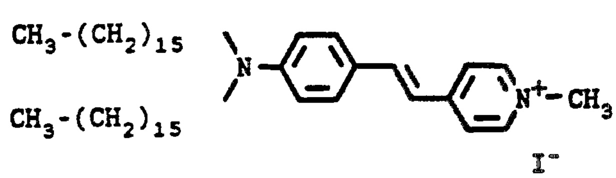 | 460 |
| 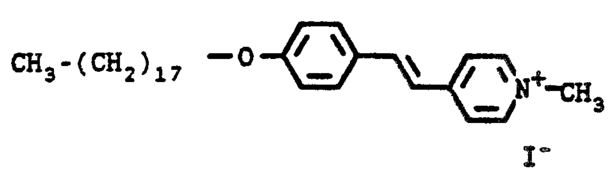 | 380 |

Schema 1

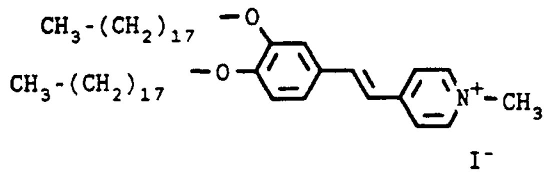

**Syntheseweg:**

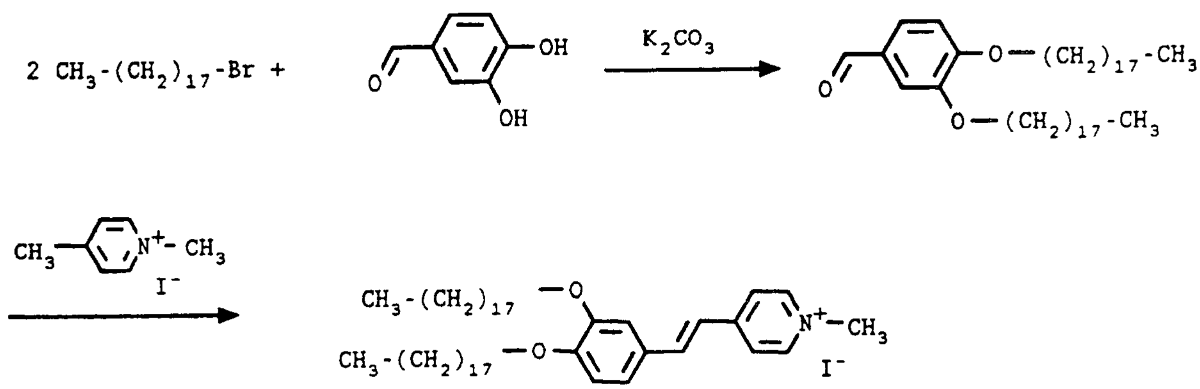

Schema 2

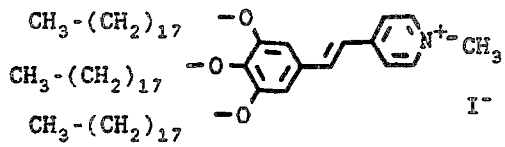

**Syntheseweg:**

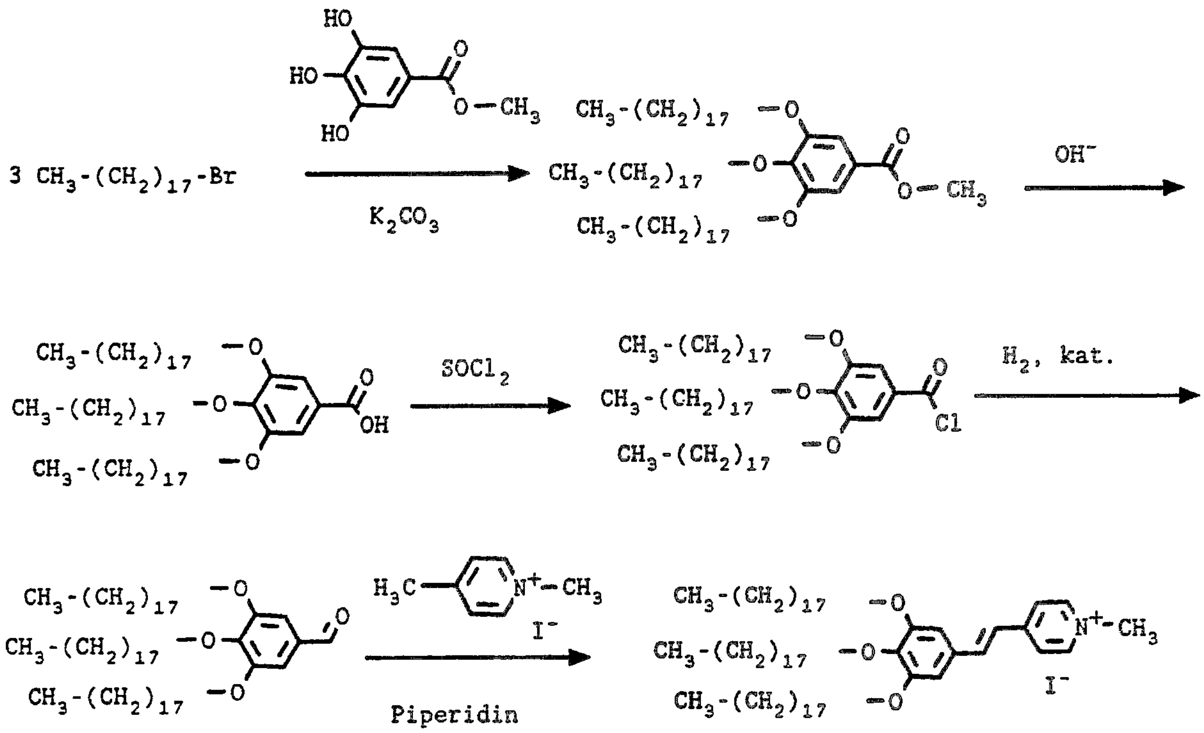

Schema 3

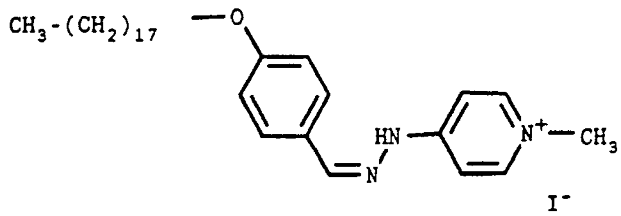

**Syntheseweg:**

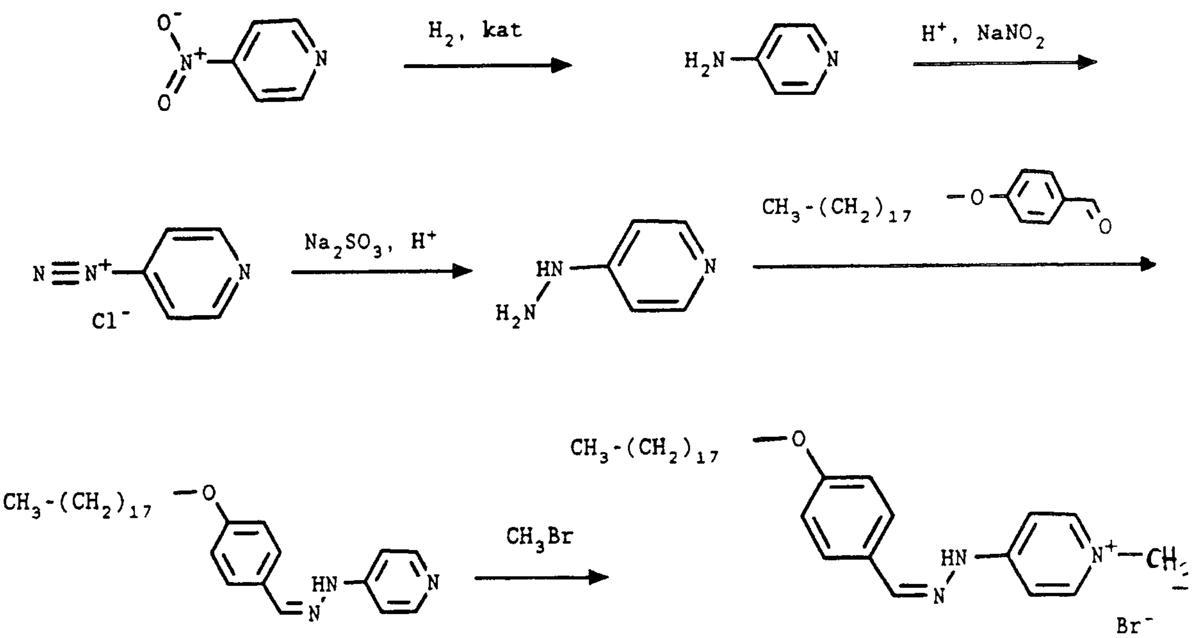

Schema 4

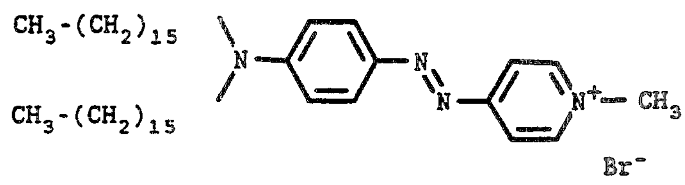

**Syntheseweg:**

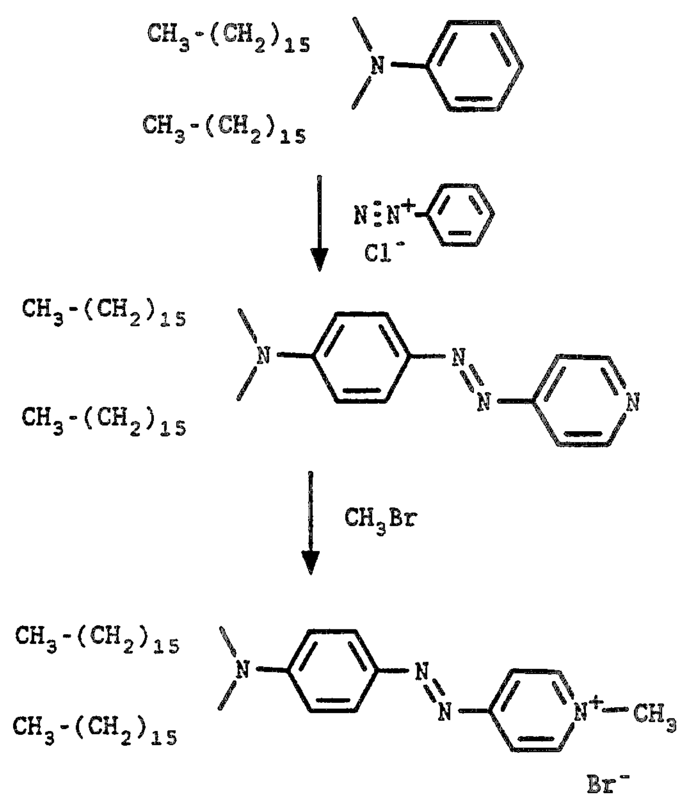

Schema 5

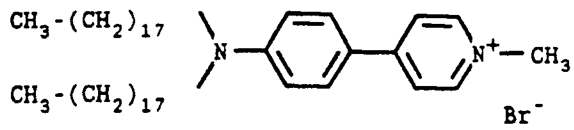

**Syntheseweg:**

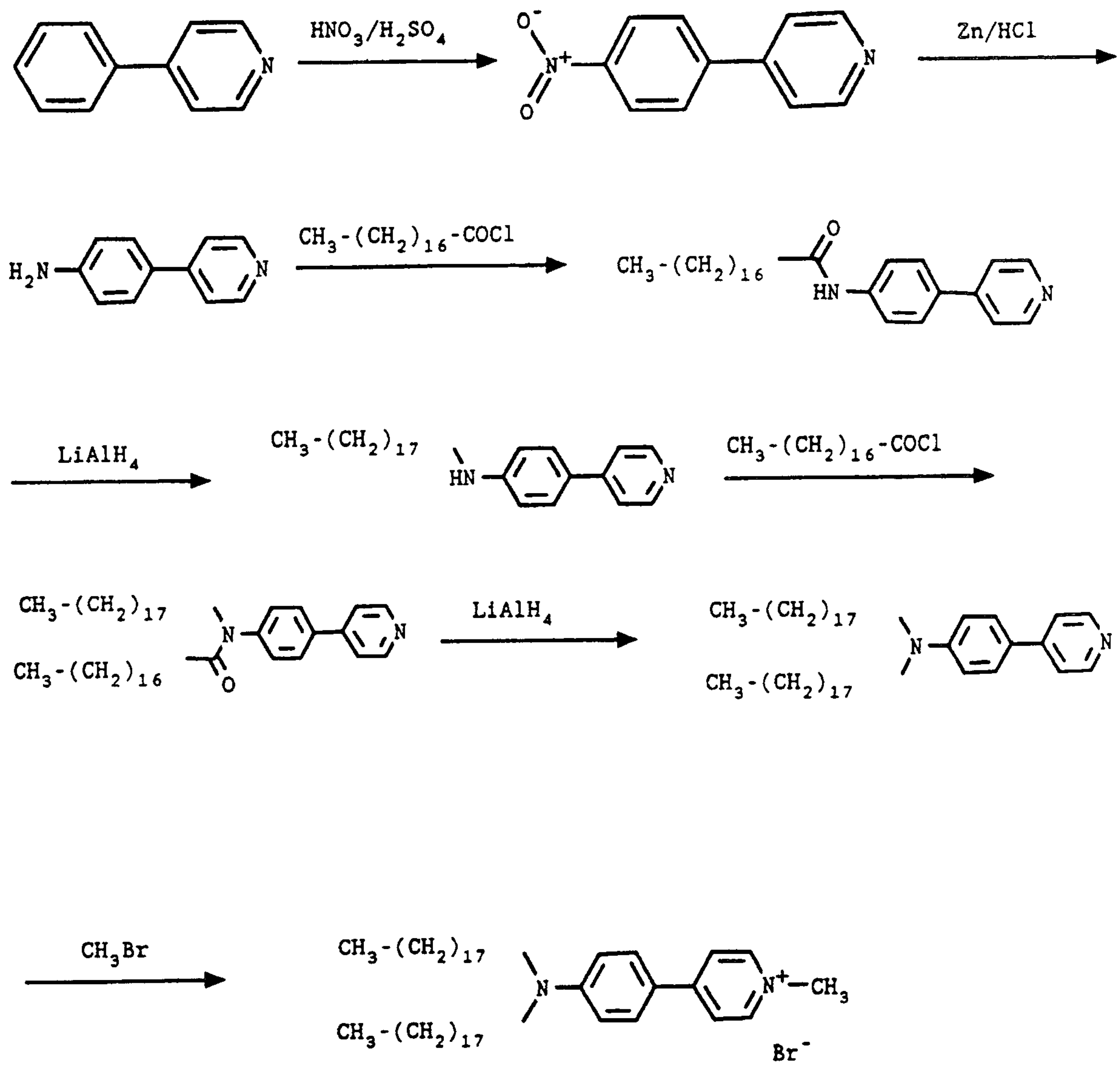


## Ansprüche

1. Schichtelement bestehend aus einem festen Schichtträger und mindestens zwei hierauf aufgebrachten dünnen Schichten regelmäßiger Struktur, die mindestens eine amphiphile Verbindung enthalten, dadurch gekennzeichnet, daß die amphiphile Verbindung die allgemeinen Formel (I) aufweist,

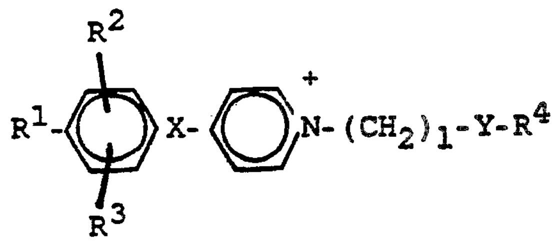

$Z^-$ (I)

wobei $R^1$ $H(cH_2)n^O$, $H(CH_2)n^S$ oder

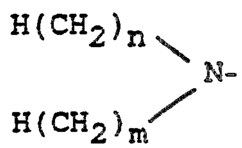

,

$R^2$ und $R^3$, unabhängig voneinander $H$, $H(CH_2)p^{O-}$, $H(CH_2)p^{S-}$ oder

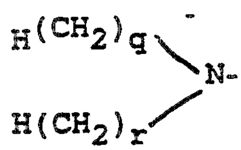

,

| | |
|---|---|
| X | eine einfache Bindung, -CH = CH-, -N = N-, -CH = N-NH-, oder -CH = N-, |
| Y | einen zweiwertigen Rest O, NH oder 5, |
| $R^4$ | $-H$, $-CO-(CH_2)_tH$, $-(CH_2)_t-H$ oder |
| $YR^4$ | $-COO^-$, $-CONH_2$, H, OH, $SO_2$ $(CH_2)u^H$, $-CO_2$ $(CH_2)t^H$ oder $-CO_2(CH_2)v^{OH}$, |
| Z | mindestens teilweise ein Polyanion |
| l | eine Zahl von 1-10, |
| n | eine Zahl von 10-25, |
| m | eine Zahl von 0-25, |
| p | eine Zahl von 10-25, |
| q | eine Zahl von 10-25, |
| r | eine Zahl von 0-25, |
| t | eine Zahl von 0-25, |
| u | eine Zahl von 1-20 und |
| v | eine Zahl von 2-10 |

bedeuten.

2. Schichtelement gemäß Anspruch 1, dadurch gekennzeichnet, daß in der amphiphilen Verbindung der allgemeinen Formel I

$R^1$ für

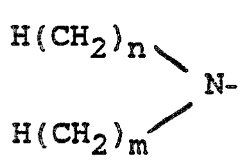

mit n = 14-24, m = 14-24 und

$R^2$ und $R^3$ für H

oder

$R^1$ und $R^2$ unabhängig voneinander für

$H(CH_2)_n$-O oder $H(CH_2)_m$-S
mit n = 14-24, m = 14-24 und
$R^3$ für H
steht.

3. Schichtelement gemäß Anspruch 1, dadurch gekennzeichnet, daß in den Schichten die Moleküle der Formel I im wesentlichen gleichmäßig und nicht-zentrosymmetrisch orientiert sind.

4. Schichtelement gemäß Anspruch 1 oder 2, dadurch gekennzeichnet, daß jeweils eine Schicht, die die Verbindung der allgemeinen Formel (I) enthält oder aus dieser besteht, abwechselt mit einer Schicht, die eine andere amphiphile Verbindung enthält oder aus dieser besteht.

5. Verfahren zur Herstellung eines Schichtelements gemäß Anspruch 1, dadurch gekennzeichnet, daß man mindestens eine amphiphile Verbindung der allgemeinen Formel I, wobei Z für ein löslich machendes Anion steht, in einem flüchtigem, mit Wasser nicht mischbaren Lösungsmittel löst, man die Lösung auf der Grenzfläche von Wasser, das ein Polyanion enthält, und Luft aufbringt, die nach Verdunsten des Lösungsmittels zurückbleibende Schicht komprimiert und nach der Langmuir-Blodgett-Technik auf einen festen Schichtträger überträgt.

6. Verfahren nach Anspruch 5, dadurch gekennzeichnet, daß man an der Oberfläche von Wasser spreitet, das Polyacrylsäure enthält.

7. Verfahren nach Anspruch 5, dadurch gekennzeichnet, daß man an der Oberfläche von Wasser spreitet, das eine Polysulfonsäure enthält.

8. Verfahren nach Anspruch 6 oder 7, dadurch gekennzeichnet, daß die Konzentration an Polysäure 0,1 mg - 1 g pro 1 Wasser beträgt.